(19) Europäisches Patentamt

European Patent Office

Office européen des brevets

(11) **EP 1 017 784 B1**

(12) **FASCICULE DE BREVET EUROPEEN**

(45) Date de publication et mention
de la délivrance du brevet:
**23.04.2003 Bulletin 2003/17**

(21) Numéro de dépôt: **98942953.5**

(22) Date de dépôt: **23.09.1998**

(51) Int Cl.⁷: **C12M 1/33**, C12N 1/06,
C12M 3/08

(86) Numéro de dépôt international:
**PCT/IB98/01475**

(87) Numéro de publication internationale:
**WO 99/015621 (01.04.1999 Gazette 1999/13)**

(54) **PROCEDE DE LYSE DE MICRO-ORGANISME**

Verfahren zur Lyse von Mikroorganismen

Process for the lysis of microorganisms

(84) Etats contractants désignés:
**AT BE CH CY DE DK ES FI FR GB GR IE IT LI LU
MC NL PT SE**

(30) Priorité: **23.09.1997 FR 9712164**

(43) Date de publication de la demande:
**12.07.2000 Bulletin 2000/28**

(73) Titulaires:
• **BIO MERIEUX
69280 Marcy l'Etoile (FR)**
• **Gen-Probe Incorporated
San Diego, CA 92121 (US)**

(72) Inventeurs:
• **SANTORO, Lyse
F-69269 Charbonnières les Bains (FR)**
• **COLIN, Bruno
F-69280 Marcy l'Etoile (FR)**
• **LOPEZ, Sophie
F-69380 Chazay d'Azergues (FR)**
• **PARIS, Cécile
F-69280 Marcy L'Etoile (FR)**
• **JAY, Corinne
F-69100 Villeurbanne (FR)**
• **CLARK DICKEY, Kathleen, Ann
Cardiff, CA 91007 (US)**

(74) Mandataire: **Guerre, Dominique et al
Cabinet Germain et Maureau,
12, rue Boileau,
BP 6153
69466 Lyon Cedex 06 (FR)**

(56) Documents cités:
EP-A- 0 284 044          EP-A- 0 288 618
EP-A- 0 317 803          EP-A- 0 341 215
EP-A- 0 770 689          FR-A- 1 576 299
US-A- 4 295 613          US-A- 4 666 850
US-A- 4 775 622          US-A- 5 254 470
US-A- 5 464 773          US-A- 5 643 767

**Description**

La présente invention concerne un procédé de lyse de micro-organisme, permettant de manière générale de rompre ce dernier, notamment la membrane d'une ou plusieurs cellules, pour libérer au moins un matériel nucléique d'intérêt, par exemple acide désoxyribonucléique (ADN) et acide ribonucléique (ARN) à traiter postérieurement, notamment analyser.

Le document US-C-5,643,767 divulgue un procédé de lyse pour séparer les ADN ou ARN de cellules dans une solution liquide. On utilise à cette fin un contenant qui contient un solvant pour extraire l'ARN ou l'ADN, une pluralité de particules ayant 0,1 à 1 mm de diamètre, et au moins une particule plus grande ayant de 3 à 5 mm de diamètre. Un mouvement est appliqué au contenant ainsi rempli, à savoir un mouvement de secousse. Il est expressément indiqué dans ce document que ce mouvement est préféré à un mouvement de rotation, tel que généré avec un mélangeur ou autre homogénéiseur, parce que avec un mouvement de rotation les cellules ne font simplement que tourner dans le même sens, et n'ont pas de collision efficace entre elles et avec les billes, pour être écrasées entre les billes.

Le document US-C-4,295,613 divulgue un appareil pour rompre des bactéries, selon lequel l'échantillon contenant les cellules est disposé dans un tube en contact avec de fines billes. Les billes, d'un diamètre de l'ordre de 70 à 110 $\mu$m, sont introduites dans le tube, avec l'échantillon à traiter et toute solution tampon nécessaire, puis on applique au contenant ou tube un mouvement d'oscillation selon un axe horizontal, afin d'obtenir les constituants sub-cellulaires en solution tels que enzymes, protéines, carbohydrates,...

Le document FR-A-1576299 divulgue un procédé pour rompre des cellules végétales et animales, selon lequel on utilise un matériau particulaire, dont la dimension des particules est de 0,05 à 1 mm de diamètre, les particules pouvant être en acier ou autre matériau analogue. Un mouvement est appliqué au mélange de l'échantillon biologique et des particules, selon un écoulement laminaire.

Le document EP-A-0317 803 divulgue un procédé pour générer des liposomes multilamellaires encapsulant un milieu aqueux. Le procédé consiste à mélanger des lipides et le milieu aqueux à encapsuler, à agiter le mélange dans un contenant en présence de particules ayant un diamètre inférieur à 3 mm, la taille préférée étant 50 à 100 $\mu$m, pour obtenir des liposomes ayant un diamètre d'environ 150 à 3000 nanomètres.

Le document EP-A-0796 917 divulgue un dispositif qui libère des particules dans un échantillon biologique contenant des cellules. Lorsque l'échantillon est agité ou soniqué, ce dispositif comprend une cloison qui retient les particules durant une durée suffisante, et ensuite les libérer et rompre les cellules, ce qui permet ainsi de rendre accessibles les acides nucléiques. Les particules retenues par la cloison, qui sont en verre, en plastique, ou en métal tel que le zirconium, peuvent avoir des formes différentes, et ont un diamètre d'environ 0,1 à 0,15 mm. Une particule est affectée à casser la cloison, et a un diamètre d'environ 1 à 4 mm, de préférence 3 mm. L'appareil de mise en mouvement alternatif est un agitateur de marque BioSpec®.

Le document EP-A-0288618 divulgue un procédé de lyse cellulaire, dont des micro-organismes, pour libérer des constituants sub-cellulaires incluant ADN et ARN en solution. L'échantillon biologique est placé dans un contenant avec des particules de tailles différentes. Le contenant est alors soumis à sonication jusqu'à ce que les cellules libèrent leurs constituants. Les ultra-sons mettent les particules en vibration à travers l'échantillon, ce qui provoque la rupture des cellules par cisaillement. Les particules sont des billes de verre de diamètre compris entre 0,05 et 1 mm. Le temps de sonication est de 10 minutes à température ambiante. L'ARN et l'ADN sont libérés de manière accessible à des sondes génétiques nucléiques d'hybridation.

Le document US-C-5,464,773 divulgue un appareil pour lyser des cellules, sans détruire les constituants subcellulaires, pour notamment libérer l'ARN et l'ADN afin d'effectuer postérieurement une hybridation. Le contenant utilisé contient deux tailles de billes en zirconium, verre, ou matière plastique ou autre. Dans un mode préféré, le contenant contient 400 $\mu$l de particules de zirconium de deux tailles et poids différents, par exemple 0,7 g et 0,1 mm de diamètre et 0,65 g et 0,5 mm de diamètre. Le mouvement appliqué au contenant est un mouvement de type vibratoire, en particulier oscillatoire.

Le document US-C-4,666,850 divulgue un appareil et un procédé pour lyser un échantillon sanguin lors de sa centrifugation. Le contenant reçevant l'échantillon sanguin contient des particules ou billes, en matière plastique ou en verre, qui doivent avoir une taille qui n'empêche pas la sédimentation des bactéries au fond du tube lors de la centrifugation.

Le document EP-A-0341215 divulgue un procédé pour produire des protéines hétérologues à partir de levures transformées génétiquement. Afin d'évaluer la quantité de protéines, il est précisé simplement que pour lyser les cellules, on applique des forces mécaniques de cisaillement en secouant l'échantillon biologique contenant les levures avec des billes de verre.

Le document EP-A-0284044 divulgue un procédé pour augmenter la production de protéines dans les levures. Il est indiqué que les cellules (levures), pour l'analyse de la quantité de protéines, sont lysées de préférence en appliquant un mouvement de type vortex à l'échantillon biologique, avec des billes de verre de diamètre 450-500 $\mu$m à vitesse maximale pendant une minute, trois fois de suite.

Le document US-C-4,775,622 divulgue un procédé pour exprimer et récupérer des protéines à partir d'une culture de levures.

Les procédés généralement utilisés pour lyser un échantillon biologique comprenant au moins un micro-organisme, spécifiquement pour libérer au moins un matériel nucléique d'intérêt, consistent essentiellement selon l'art antérieur en ce que:

- on dispose dans un contenant un échantillon biologique en milieu liquide, comprenant le micro-organisme à lyser,
- on dispose dans ledit contenant au moins un matériau particulaire, relativement dur, et substantiellement inerte par rapport au matériau nucléique,
- et on soumet le mélange de l'échantillon biologique et du matériau particulaire essentiellement à un mouvement alternatif, d'amplitude et/ou fréquence variable s'il s'agit d'un mouvement régulier, selon la technique ou l'équipement retenu pour la mise en mouvement.

Ces procédés utilisés présentent certains inconvénients. Ils ne sont pas suffisamment efficaces, notamment la lyse cellulaire s'avère insuffisante pour libérer le matériel nucléique en quantité et qualité.

De plus, ils ne permettent pas toujours de lyser des cellules réputées résistantes à la lyse, notamment les cellules des bactéries à Gram+, par exemple celles de Mycobactéries.

De même, la mise en oeuvre de ces procédés nécessite souvent l'ajout de réactifs supplémentaires tels que par exemple des enzymes et/ou des détergents.

Selon la présente invention, on a trouvé que, contrairement à l'enseignement divulgué dans le brevet US 5,643,767, un mouvement rotatoire de type Vortex était particulièrement adapté, pour lyser des micro-organismes et libérer directement le matériau nucléique d'intérêt, à la condition de choisir certains paramètres pour ce mouvement, en relation notamment avec le contenant.

L'invention a donc pour objet un procédé de lyse complète, efficace et simple d'un échantillon biologique comprenant au moins un micro-organisme, sans réactif et/ou étape opératoire supplémentaire au cours de la mise en oeuvre du procédé.

La premier objet de la présente invention est donc un procédé de lyse d'un échantillon biologique comprenant au moins un micro-organisme de type bactérie, pour libérer au moins un matériel nucléique d'intérêt appartenant audit micro-organisme, selon lequel:

- on dispose dans un contenant ledit échantillon biologique en milieu liquide,
- on dispose dans ledit contenant au moins un matériau particulaire, relativement dur, et substantiellement inerte par rapport au matériel nucléique,
- on soumet le mélange de l'échantillon biologique et du matériau particulaire à un mouvement,

caractérisé en ce que, en combinaison:

le mouvement choisi est de type vortex, et répond aux conditions suivantes:

- le matériau particulaire est constitué de billes de diamètre compris entre 90 et 150 $\mu$m, et
- le volume apparent des billes , Vb, et le volume de l'échantillon liquide, Ve, sont liés par la relation Ve = $\alpha$.Vb, avec $\alpha$ compris entre 1,4 et 10 lorsque le contenant est de forme tubulaire, et $\alpha$ est inférieur ou égal à 2,1 lorsque le contenant est de forme disque,

moyennant quoi, sans ajout de réactif et/ou étape opératoire supplémentaire, on libère directement dans le milieu liquide le matériel nucléique à l'état natif et accessible à tout réactif.

Un second objet selon l'invention est un procédé de lyse d'un échantillon biologique comprenant au moins un micro-organisme de type levure, pour libérer au moins un matériel nucléique d'intérêt appartenant audit micro-organisme, selon lequel:

- on dispose dans un contenant ledit échantillon biologique en milieu liquide,
- on dispose dans ledit contenant au moins un matériau particulaire, relativement dur, et substantiellement inerte par rapport au matériel nucléique,
- on soumet le mélange de l'échantillon biologique et du matériau particulaire à un mouvement,

caractérisé en ce, que en combinaison:

le mouvement choisi est de type vortex, et répond aux conditions suivantes:

- le matériau particulaire est constitué de billes ayant un diamètre 500 μm, et
- le volume apparent des billes , Vb, et le volume de l'échantillon liquide, Ve, sont liés par la relation Ve = α.Vb, avec α compris entre 1,4 et 10 lorsque le contenant est de forme tubulaire, et α inférieur ou égal à 2,1 lorsque le contenant est de forme disque,

moyennant quoi, sans ajout de réactif et/ou étape opératoire supplémentaire, on libère dans le milieu liquide le matériel nucléique à l'état natif et accessible à tout réactif.

Par le procédé selon l'invention, on obtient le matériel nucléique à l'état natif, ce qui veut dire qu'il est pour l'essentiel non détérioré, par exemple non dénaturé, ou qu'il conserve pratiquement toutes les caractéristiques ou propriétés qu'il avait au sein de la cellule ou organisme dont il est extrait.

Par ailleurs, le procédé selon l'invention est suffisamment efficace pour permettre la lyse d'un seul micro-organisme, ce qui pour beaucoup de procédés d'analyse biologique, notamment pour les techniques d'amplification ou d'analyse par hybridation de sonde(s) nucléique(s) revêt un intérêt particulier.

S'agissant du matériel nucléique, la présente invention apporte l'avantage déterminant de libérer ledit matériel de manière directement accessible pour tout protocole postérieur le concernant, par exemple une ou des amorces d'amplification, une ou des sondes d'hybridation...

Par conséquent, selon la présente invention, l'échantillon biologique lysé obtenu, peut être traité directement, c'est à dire sans étape opératoire intermédiaire, ou sans réactif ajouté, selon tout protocole opératoire en relation avec le matériel nucléique d'intérêt libéré, tel que amplification, analyse, etc., avec tous réactifs nucléiques appropriés, ou autres.

Dans un mode de réalisation préféré selon le premier objet de l'invention, le matériau particulaire est constitué de billes de diamètre de 100 μm, pour un micro-organisme du type bactérie.

Dans un autre mode de réalisation selon le premier ou second objet de l'invention, le mouvement de type vortex répond en outre à la relation suivante:

$$Vb < Ve < Vc,$$

selon laquelle

$$Vc = \beta.Ve,$$

et

β est supérieur ou égal à 2,5, avec Vc le volume utile du contenant.

Les contenants que l'on peut utiliser selon l'invention peuvent être de formats différents, par exemple de forme tubulaire ou de forme disque.

On préfère que le contenant soit de forme tubulaire, de préférence à fond en forme de U. Dans ce mode de réalisation préféré, β est un nombre compris entre 2,5 et 30.

Dans encore un autre mode de réalisation selon l'invention, les contenants sont de forme disque et de forme tubulaire à fond en forme de U (comme par exemple de type Falcon®) ou à fond tronconique (comme par exemple de type Eppendorf®).

Lorsque le contenant est sous forme disque, α est un nombre inférieur ou égal à 2,1, de préférence inférieur ou égal à 1,4 et β est un nombre supérieur ou égal à 9,3.

On préfère que le contenant soit un tube à fond tronconique tel qu'un tube Eppendorf, α étant compris entre 1,4 et 10, de préférence entre 1,4 et 3,3, et β étant compris entre 2,5 et 30, de préférence entre 2,5 et 15, de préférence encore entre 3,75 et 15.

On préfère encore que le contenant soit un tube à fond en U, tel qu'un tube Falcon, α étant compris entre 1,4 et 10, de préférence entre 1,4 et 3,3, de préférence encore est égal à 3,3, et β étant compris entre 3 et 30, de préférence entre 12 et 30, de préférence encore est égal à 20.

Dans encore un autre mode de réalisation selon l'invention, le matériau particulaire comprend d'autres billes ayant un diamètre supérieur à celui desdites billes. On préfère que le contenant comprenne jusqu'à 16, de préférence 10 autres billes. On préfère encore plus que ces autres billes aient un diamètre de 2 à 3 mm.

Cette caractéristique complémentaire du procédé selon l'invention permet, pour une efficacité équivalente, en particulier de diminuer ou limiter le temps requis pour la lyse de l'échantillon biologique.

**EP 1 017 784 B1**

Cette caractéristique complémentaire permet aussi de rompre des échantillons biologiques complexes, tels que tissus vivants, avant la lyse proprement dite.

Dans encore un autre mode de réalisation selon l'invention, on peut ajouter à l'échantillon biologique une substance anti-mousse à une concentration finale de 0,01 à 1% en volume, de préférence de 0,5 à 1% du volume de l'échantillon liquide, Ve.

On peut encore, selon un autre mode de réalisation, ajouter à l'échantillon biologique un détergent de type anionique à une concentration finale de 2,5% du volume de l'échantillon liquide, Ve.

Dans un mode de réalisation préféré selon le premier objet de l'invention, le temps de mise en mouvement de type vortex est au moins égal à 10 secondes, de préférence au moins 20 secondes, avantageusement entre 1 et 5 minutes.

Le temps de vortex est adapté de manière à obtenir un pourcentage de lyse au moins supérieur ou égale à 20%.

On préfère encore, selon les spécificités du protocole de lyse utilisé, notamment lorsqu'on ajoute des billes de plus gros diamètre, que le temps de mise en mouvement de type vortex soit 2 minutes.

Dans un mode de réalisation préféré selon le second objet de l'invention, le temps de mise en mouvement de type vortex est compris entre 8 et 20 minutes.

Etant donné que le lysat obtenu permet directement de détecter et/ou quantifier et/ou amplifier le matériel nucléique d'intérêt, la présente invention concerne également un procédé de traitement d'un échantillon biologique comprenant un micro-organisme comportant un matériel nucléique d'intérêt, ledit procédé comprenant:

a) une étape de lyse, selon laquelle:

- on dispose dans un contenant ledit échantillon biologique en milieu liquide,
- on dispose dans ledit contenant au moins un matériau particulaire, relativement dur, et substantiellement inerte par rapport au matériel nucléique,
- et on soumet le mélange de l'échantillon biologique et du matériau particulaire à une mise en mouvement du type vortex, selon laquelle en combinaison:

- les billes ont un diamètre compris entre 90 et 150 µm pour un micro-organisme de type bactérie, et 500 µm pour un micro-organisme de type levure,
- le volume apparent des billes, Vb, et le volume de l'échantillon liquide, Ve, sont liés par la relation $Ve = \alpha Vb$, avec $\alpha$ compris entre 1,4 et 10 lorsque le contenant est de forme tubulaire, et $\alpha$ est inférieur ou égal à 2,1, lorsque le contenant est en forme de disque,

b) on soumet l'échantillon biologique lysé, avec ou sans le matériau particulaire ayant servi à la lyse, directement à un protocole opératoire concernant spécifiquement le matériel nucléique d'intérêt par exemple amplification et/ ou détection et/ou quantification nucléique.

Ainsi, par exemple après amplification, on peut détecter au moins $1.10^2$ cellules/ml.

Un troisième objet selon l'invention est un contenant à usage unique, pour la mise en oeuvre d'un procédé décrit précédemment, caractérisé en ce qu'il comprend une charge d'un matériau particulaire adaptée, en fonction d'un volume prédéterminé de l'échantillon biologique, à la mise en oeuvre directe du procédé décrit précédemment.

Par "échantillon biologique", on entend tout échantillon, prélèvement ou fraction d'un matériel biologique, simple ou complexe, par exemple un tissu vivant ou un liquide ou fluide corporel. Cet échantilon biologique contient au moins un micro-organisme à lyser, avec ou sans traitement préalable de la structure comprenant ledit micro-organisme, par exemple destruction de l'organisation du tissu.

Par "micro-organisme", on entend tout matériau biologique, comprenant naturellement une membrane ou enveloppe fermée, un ou plusieurs constituants biologiques d'intérêt, sub-cellulaires, renfermés à l'intérieur de ladite membrane ou enveloppe, à savoir nucléiques (ADN ou ARN). A titre d'exemple, on peut citer bien entendu les micro-organismes procaryotes, tels que les bactéries et archaebactéries, les micro-organismes eucaryotes tels que les cellules végétales ou animales, les levures et les champignons, également différents tissus vivants, et par extension des particules virales.

Par le terme " lyse ", on entend tout processus permettant de rompre la membrane ou l'enveloppe précitée pour libérer le matériel nucléique d'intérêt, sous forme totale ou partielle.

Les figures et les exemples qui suivent permettent d'illustrer l'objet de l'invention, mais ne limitent en rien la portée de celle-ci.

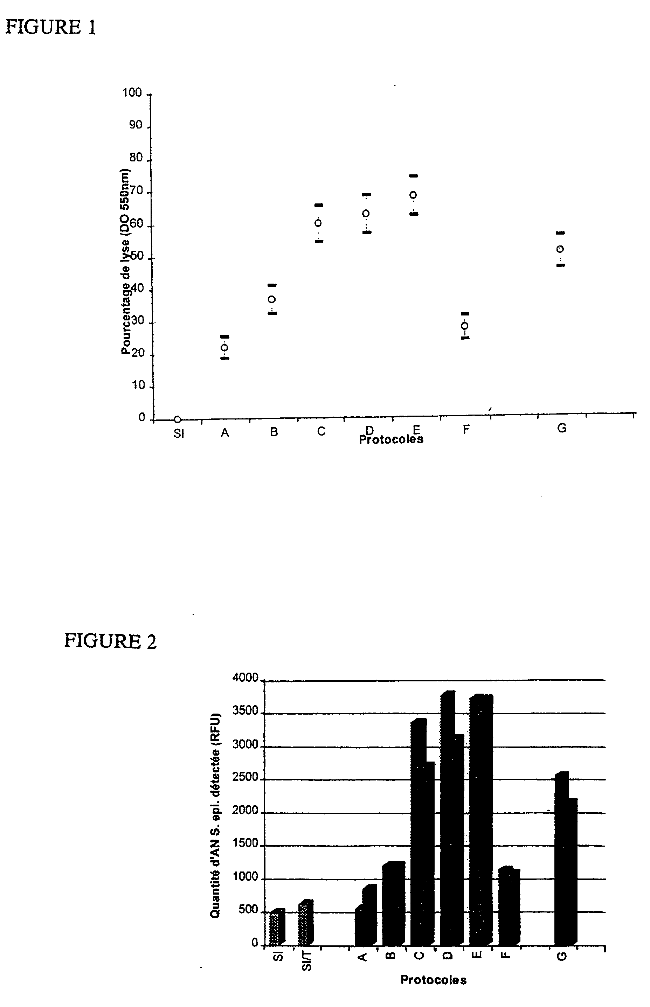

La figure 1 illustre le pourcentage de lyse (en ordonnée) en fonction du diamètre des billes (en abscisse), et de la composition du mélange (nature et proportion des billes).

" SI " signifie " solution initiale " et représente le témoin qui n'a pas été lysé.

A : Billes de verre de 1 à 50 $\mu$m
B : Billes de verre de 50 à 100 $\mu$m
C : Billes de verre de 90 à 150 $\mu$m
D : Billes de verre 100 $\mu$m
E : Billes de zirconium 100 $\mu$m
F : Billes de zirconium 500 $\mu$m
G : Mélange 50/50 de billes de zirconium de 100 et 500 $\mu$m.

La figure 2 illustre la quantité d'acides nucléiques de *S.epidermidis* détectée (en ordonnée) en fonction du diamètre des billes (en abscisse).
" SI " signifie " solution initiale " et représente le témoin qui n'a pas été lysé.

A : Billes de verre de 1 à 50 $\mu$m
B : Billes de verre de 50 à 100 $\mu$m
C : Billes de verre de 90 à 150 $\mu$m
D : Billes de verre 100 $\mu$m
E : Billes de zirconium 100 $\mu$m
F : Billes de zirconium 500 $\mu$m
G : Mélange 50/50 de billes de zirconium de 100 et 500 $\mu$m.

La figure 3 illustre le pourcentage de lyse (en ordonnées) en tube Eppendorf en fonction du coefficient a (en abscisse).
La figure 4 illustre le pourcentage de lyse (en ordonnées) en tube Falcon en fonction du coefficient a (en abscisse).
La figure 5 illustre le pourcentage de lyse (en ordonnées) dans un contenant en forme de disque en fonction du coefficient a (en abscisse).
La figure 6 illustre le pourcentage de lyse (en ordonnées) en tube Eppendorf en fonction du coefficient b (en abscisse).
La figure 7 illustre le pourcentage de lyse (en ordonnées) en tube Falcon en fonction du coefficient b (en abscisse).
La figure 8 illustre le pourcentage de lyse (en ordonnées) dans un contenant en forme de disque en fonction du coefficient b (en abscisse).
La figure 9 illustre le pourcentage de lyse (en ordonnées) en fonction de la géométrie du contenant (en abscisse).

A : tube Eppendorf à fond en forme de V, Vc = 1,5 ml
B : cuvette de spectrophotométrie à fond plat, Vc = 5 ml
C : tube à fond plat, Vc = 30 ml
D : tube à fond plat, Vc = 60 ml
E : bouteille de verre à fond plat, Vc = 8 ml
F : bouteille de verre à fond plat, Vc = 25 ml
G : tube en polystyrène à fond en forme de U, Vc = 6 ml
H: tube en polystyrène à fond en forme de U, Vc = 14 ml
I : tube en polystyrène à fond en forme de U, Vc = 22 ml
J : tube en polypropylène à fond en forme de U, Vc = 6 ml
K : tube en polypropylène à fond en forme de U, Vc = 14 ml
L : tube en polypropylène à fond en forme de U, Vc = 22 ml
M : tube en polypropylène à fond en forme de U, Vc = 4 ml.

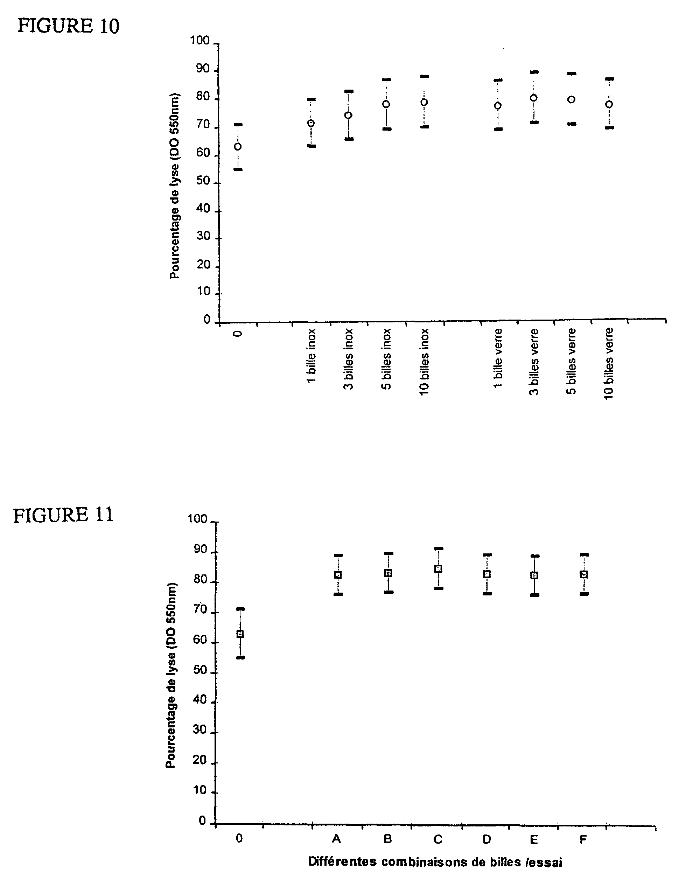

La figure 10 illustre le pourcentage de lyse (en ordonnées) en fonction de différentes billes ajoutées (en abscisse).
La figure 11 illustre le pourcentage de lyse (en ordonnées) en fonction de combinaisons de différentes billes ajoutées (en abscisse).

A : 10 billes inox de diamètre 2 mm
B : 10 billes de verre de diamètre 3 mm

C : 5 billes inox et 5 billes de verre de diamètres respectifs 2 et 3 mm
D : 4 billes inox et 4 billes de verre de diamètres respectifs 2 et 3 mm
E : 5 billes inox et 3 billes de verre de diamètres respectifs 2 et 3 mm
F : 3 billes inox et 5 billes de verre de diamètres respectifs 2 et 3 mm.

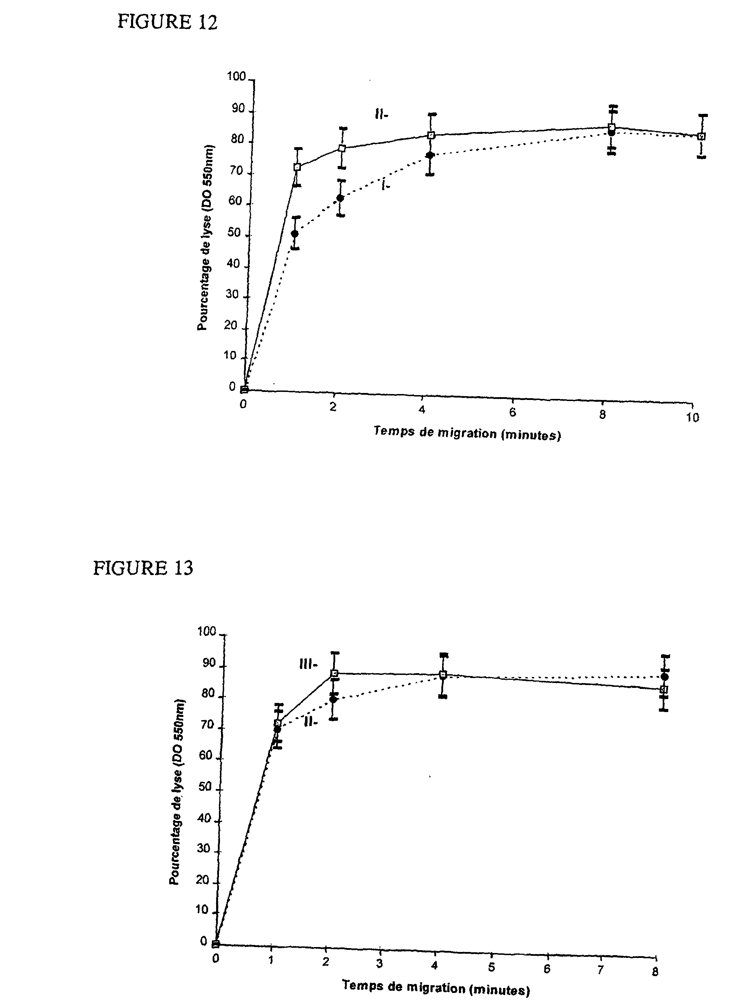

La figure 12 illustre le pourcentage de lyse (en ordonnées) en fonction du temps de mise en mouvement de type vortex (en abscisse) pour les protocoles I et II de l'exemple 5.

La figure 13 illustre le pourcentage de lyse (en ordonnées) en fonction du temps de mise en mouvement de type vortex (en abscisse) pour les protocoles II et III de l'exemple 5.

Afin de déterminer l'efficacité du procédé de lyse selon l'invention, on a mis au point un protocole test général d'analyse du lysat obtenu en mesurant le pourcentage de lyse.

Ce protocole général est décrit ci-après.

Des bactéries à paroi Gram+ *Staphylococcus epidermidis* (bioMérieux référence API N° 8149310) cultivées en milieu liquide BCC (Bouillon Coeur Cervelle) sont centrifugées à 2500 tours/minute pendant 10 minutes à 25°C, avant d'être resuspendues indifféremment en milieu BCC ou en tampon de lyse à la concentration $5.10^9$ cellules /300 µl. La composition du tampon de lyse est la suivante: 30mM Tris-Hcl, 5mM EDTA, 100 mM NaCL, pH 7.2. 300 µl de la suspension sont ajoutés dans un tube contenant préalablement un volume défini de billes de verre. Le tube fermé est mis en mouvement de type vortex pendant 2 minutes, à puissance maximale de l'appareil (Reax 2000, Heidolph). La suspension bactérienne ainsi traitée est conservée dans la glace avant analyse.

Le pourcentage de lyse est déterminé immédiatement après recueil de l'échantillon, par mesure de la densité optique à 550 nm. Le pourcentage de lyse est égal au rapport de la valeur de DO à 550 nm de la solution bactérienne après vortex sur la valeur de DO à 550 nm avant vortex.

La qualité des acides nucléiques libérés par l'étape de vortex est vérifiée sur gel d'agarose 0,8%. 10 µl du lysat sont déposés par puits, la migration est réalisée sous voltage constant (150V) et le gel est coloré au bromure d'éthidium (BET) avant observation sous rayonnement ultra-violet.

La quantité d'acides nucléiques libérés par l'étape de vortex est déterminée par détection spécifique selon la technique d'hybridation dite sandwich en utilisant l'appareil Vidas® commercialisé par bioMérieux (France). Des sondes oligonucleotidiques de capture et de détection spécifiques des acides nucléiques de *S. epidermidis* (voir le brevet EP 0632269) ont été choisies. Les oligonucleotides de capture et de détection ont respectivement pour séquence : 5'-GACCACCTGTCACTCTGTCCC-3' (SEQ ID N°:1) et 5'GGAAGGGGAAAACTCTATCTC-3' (SEQ ID N°: 2). La sonde de détection est marquée par couplage avec la phosphatage alcaline (PA). L'hybridation spécifique de ces sondes avec les acides nucléiques libérés dans le lysat est fonction de la quantité d'acides nucléiques présents, mais aussi de leur accessibilité pour les sondes utilisées.

Deux protocoles d'amplification spécifiques des molécules d'ADN et d'ARN de *S. epidermidis* ont été réalisés à partir des lysats recueillis pour vérifier si les acides nucléiques libérés par vortex peuvent être amplifiés : un protocole PCR pour l'amplification de l'ADN et un protocole NASBA pour l'amplification de l'ARNrl6S.

*Protocole* PCR : la technique de PCR suivie est celle décrite par Goodman dans *PCR* stratégies, *Ed* : Innis, Gelford et Sninsky Académie press 1995, pp 17-31. Deux amorces d'amplification ont été utilisées, elles présentent les séquences suivantes

**Amorce 1: 5'-ATCTTGACATCCTCTGACC-3' SEQ ID N°:3**

**Amorce 2: 5'-TCGACGGCTAGCTCCAAAT-3' SEQ ID N°:4**

Les cycles de température suivants ont été utilisés

| | | |
|---|---|---|
| 1 fois | 3 minutes | 94°C |
| | 2 minutes | 65°C |
| 35 fois | 1 minute | 72°C |
| | 1 minute | 94°C |
| | 2 minutes | 65°C |
| 1 fois | 5 minutes | 72 °C |

*Protocole* NASBA : la technique de NASBA suivie est celle décrite par Van der Vliet *et al*., *J.* Gen. Microbiol. 1993, 139 : 2423. Deux amorces d'amplification ont été utilisées, elles présentent les séquences suivantes :

**Amorce 1: 5'-GGTTTGTCACCGGCAGTCAACTTAGA-3'**

**Amorce 2: 5'-TCGAAGCAACGCGAAGAACCTTACCA-3'**

10 μl de lysat ou 5 μl de lysat sont utilisés pour chaque essai PCR et NASBA, respectivement. Les amplicons produits par PCR sont observés sur gel d'agarose 0.8% et quantifiés sur Vidas selon le protocole décrit ci-dessus. Les amplicons produits après NASBA sont détectés et quantifiés sur microplaque par hybridation avec une sonde de capture et une sonde de détection spécifique de *S. epidermidis*, selon la méthode décrite par P. Cros *et al.*, Lancet 1992, 240 : 870. La sonde de détection est couplée à la peroxidase de Raifort (HRP). Les deux sondes présentent les séquences suivantes :

**Sonde de capture: 5'-GATAGAGTTTTCCCCTTC-3' (SEQ ID N°: 7)**

**Sonde de détection: 5'-GACATCCTCTGACCCCTC-3' (SEQ ID N°: 8)**

Exemple 1 Influence du diamètre des billes et du coefficient a sur l'efficacité de la lyse par vortex

a- Influence du diamètre des billes.

Le protocole de lyse a été réalisé comme décrit ci-dessus à partir d'une suspension initiale de *Staphylococcus epidermidis (5.10⁹ cellules/300 μl)* en présence de 90 μl de différents mélanges de billes caractérisées par des diamètres différents, pendant 2 minutes par essai. Les diamètres testés sont compris entre 1 et 500 μm; les mélanges testés sont les suivants :

A : microbilles de verre de diamètre compris entre 1 et 500 μm,
B : microbilles de verre de diamètre compris entre 50 et 100 μm,
C : microbilles de verre de diamètre compris entre 90 et 150 μm,
D : mélange homogène de billes de diamètre 100 μm,
E : mélange homogène de billes de zirconium de diamètre 100 μm
F : mélange homogène de billes de zirconium de diamètre 500 μm
G : mélange (50/50 v/v) de billes de zirconium de diamètre 100 et 500 μm.

Comme l'indique la figure 1, la lyse cellulaire obtenue est la plus efficace en présence de billes de diamètre compris entre 90 et 150 μm, et de préférence égal à 100 μm. Les mesures de densité optique à 550nm des échantillons recueillis après 2 minutes de vortex dans ces conditions indiquent que le pourcentage de lyse de *S. epidermidis* est compris entre 60 et 70% avec ce diamètre de billes. Seule des différences négligeables de pourcentage de lyse sont observées en fonction de la nature des billes (verre ou zirconium) de même diamètre. Par contre l'utilisation de billes de diamètre inférieur ou supérieur à 90 jusqu'à 150 μm, telles les mélanges de billes de diamètre 1 à 50 μm, 50 à 100 μm, 100 à 500 μm, ou le mélange homogène de billes de diamètre 500 μm entraîne une diminution du pourcentage de lyse. L'analyse sur gel d'agarose 0,8% des acides nucléiques libérés dans les lysats, présentée dans la figure 2, montre que les molécules d'ADN et d'ARNr sont bien conservées puisque leur profil de migration sur gel d'agarose 0,8% est le même que celui des molécules d'ADN et ARNr bactériens purifiés et commercialisés. Les acides nucléiques relargués ont également le même profil de migration quel que soit le diamètre des billes utilisées pour la lyse cellulaire. Les acides nucléiques libérés sont également détectables par analyse Vidas dont le protocole est décrit ci-dessus. Toujours d'après la figure 2, la quantité d'acides nucléiques libérés dans les lysats mesurée par analyse Vidas reflète le pourcentage de lyse obtenu. En parallèle, il a été démontré que l'ADN ou l'ARNr 16S de chaque lysat peut être amplifié par protocole PCR ou NASBA spécifiques de *S. epidermidis*, qui sont décrits dans la partie protocole test general ci-dessus.

b- Influence du coefficient $\alpha$

- tube Eppendorf

Le protocole de lyse a été réalisé en présence de billes de diamètre 100 μm, pendant 2 minutes. Des tubes Eppendorfs identiques ont été utilisés pour tous les essais. Le volume de la suspension bactérienne a été fixé à 300 μl /tube pour chaque essai, et le volume de billes a varié entre 1 et 300 μl/tube. Soit Vb, le volume apparent des billes et Ve, le volume de l'échantillon liquide tel que Vb < Ve avec Ve = $\alpha$.Vb avec $\alpha$ étant un nombre réel positif différent de 0. Le coefficient $\alpha$ a varié de 1 à 300. Après l'étape de vortex, les échantillons recueillis sont analysés par mesure de densité optique à 550 nm, les acides nucléiques libérés dans le lysat sont observés sur gel d'agarose 0,8% et quantifiés par analyse Vidas.

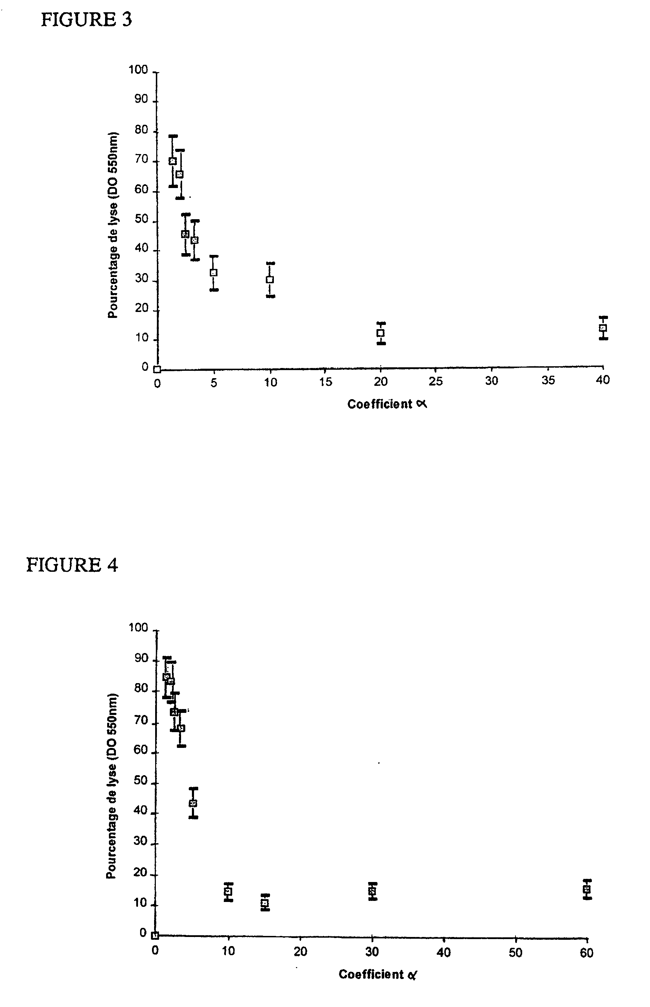

La figure 3 indique que la lyse de *S. epidermidis* est efficace pour des valeurs du coefficient $\alpha$ comprises entre 1,4 et 10 : le pourcentage de lyse est compris entre 30 et 70%. Pour des valeurs de $\alpha$ < 1,4, le volume de billes étant trop important, le volume mort de la suspension cellulaire augmente de façon telle qu'il est impossible de récupérer le volume initial de la suspension cellulaire. Pour des valeurs de $\alpha$ > 10, le volume de billes est faible et la probabilité de rencontre et de choc mécanique entre billes et cellules est réduite. De préférence la lyse cellulaire est plus efficace pour des valeurs de $\alpha$ comprises entre 1,4 et 3,3 : le pourcentage de lyse étant compris entre 40 et 70%. L'analyse sur gel d'agarose 0,8% du lysat montre que les profils de migration des acides nucléiques libérés ne sont pas modifiés, quelles que soient les valeurs de $\alpha$. La quantité d'acides nucléiques mesurée par analyse Vidas est directement corrélée au pourcentage de lyse.

- tube Falcon

Une même étude a été réalisée en remplaçant les tubes Eppendorf par des tubes Falcon en polypropylène (Vc = 6ml). La figure 4 indique que la lyse de *S. epidermidis est* efficace pour des valeurs du coefficient $\alpha$ comprises entre 1,4 et 10 : le pourcentage de lyse est supérieur à 20%. De préférence, la lyse cellulaire est plus efficace pour des valeurs de $\alpha$ comprises entre 1,4 et 3,3 : le pourcentage de lyse est compris entre 65 et 85%.

-forme disque

On a utilisé une carte de dimensions 1 = L = 8,5cm et h = 0.4 cm. Un ou plusieurs trous, définis comme des puits, sont réalisés de hauteur constante et égale à la hauteur de la carte (soit 0.4 cm) et de diamètre constant. Ces puits sont recouverts d'un film de chaque côté, et chaque puits est rempli d'un mélange de billes de diàmètre 100 μm de volume apparent Vb, de 10 billes de fer de diamètre 2 mm et d'une suspension cellulaire de volume Ve. La carte ainsi définie est maintenue sur le vortex.

Le protocole de lyse a été réalisé pendant deux minutes. Des cartes telles que décrites ci-dessus avec un diamètre de 30 cm (Vc = 2,8 ml/puits) ont été utilisées pour chaque essai. Le volume de la suspension bactérienne a été fixée à 700 μl /puits et le volume de billes a varié de 90 à *510* μl/puits. Le coefficient $\alpha$ a varié de 1,4 à 7,8. Après l'étape de vortex, le pourcentage de lyse des échantillons recueillis a été analysé par mesure de densité optique à 550 nm, les acides nucléiques libérés ont été observés sur gel d'agarose et quantifiés par analyse Vidas.

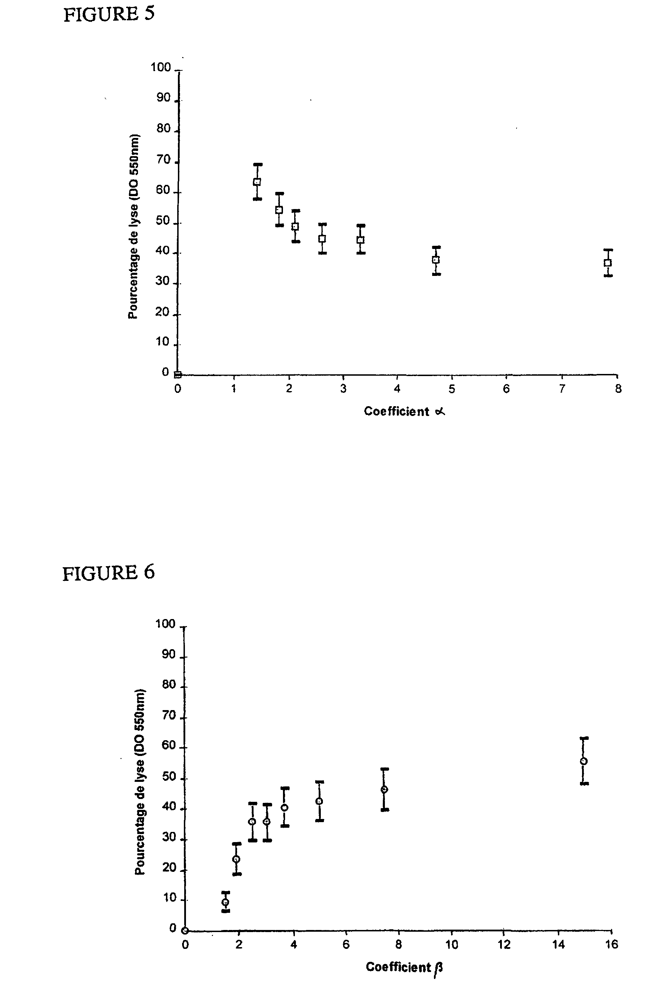

La figure 5 indique le pourcentage de lyse obtenu en fonction du coefficient $\alpha$. La lyse de *S.epidermidis* est efficace avec un contenant format "carte" pour des valeurs du coefficient inférieures ou égales à 2,1 : lorsque $\alpha$ est compris entre 1,4 et 2,1, le pourcentage de lyse est compris entre 45% et 75%. De préférence, la valeur optimale du coefficient devrait être inférieure à 1,4 car même pour des valeurs de $\alpha$ s'approchant de 1,4, les valeurs de pourcentage de lyse n'arrivent pas à un plateau. L'analyse sur gel d'agarose 0,8% du lysat montre que les profils de migration nucléiques libérés ne sont pas modifiés quelles que soient les valeurs de $\alpha$. La quantité d'acide nucléique mesurée par analyse Vidas est directement corrélée au pourcentage de lyse.

Exemple 2 : influence du coefficient $\beta$ sur l'efficacité de la lyse par vortex

- Tube Eppendorf

Le protocole de lyse a été réalisé en présence d'un rapport constant entre le volume apparent de billes et le volume de l'échantillon liquide ($\alpha$ = Ve/Vb = 3,3) pendant 2 minutes. Le volume de la suspension bactérienne a varié entre 100 μl et 1 ml. Soit Ve, le volume de l'échantillon liquide et Vc, le volume utile ou disponible du contenant tel que Ve < Vc et Vc = $\beta$.Ve avec $\beta$ étant un nombre réel positif supérieur à 0, le coefficient $\beta$ a varié de 1,5 à 15. Les expériences ont été réalisées en tube Eppendorf, soit Vc = 1,5ml. Après l'étape de vortex, les échantillons recueillis sont analysés par mesure de densité optique à 550 nm, les acides nucléiques dans le lysat sont observés sur gel d'agarose 0,8% et analysés par analyse Vidas.

Sur la figure 6 est indiqué le pourcentage de lyse obtenu en fonction du coefficient β. La lyse de *S. epidermidis est* plus efficace pour des valeurs du coefficient β compris entre 2,5 et 15 : le pourcentage de lyse est supérieur à 30%. Pour des valeurs de β inférieures à 2,5, les volumes de billes et d'échantillon liquide sont trop importants; ils limitent le mouvement des billes dans l'échantillon et le volume mort de l'échantillon augmente. Pour des valeurs de β supérieures à 15, le volume de l'échantillon est trop faible pour permettre une récupération complète du volume initial de la suspension cellulaire pour α = 3,3. De préférence, l'efficacité de lyse cellulaire est meilleure pour des valeurs de β comprises entre 3,75 et 15 : le pourcentage de lyse est compris entre 35 et 60%; de préférence pour des valeurs comprises entre 7,5 et 15 (40 à 60% de lyse). L'analyse sur gel d'agarose 0,8% des lysats montre que les profils de migration des acides nucléiques libérés sont les mêmes quelles que soient les valeurs de β. Les quantités d'acides nucléiques mesurées par analyse Vidas sont directement corrélées aux pourcentages de lyse obtenus.

- tube Falcon

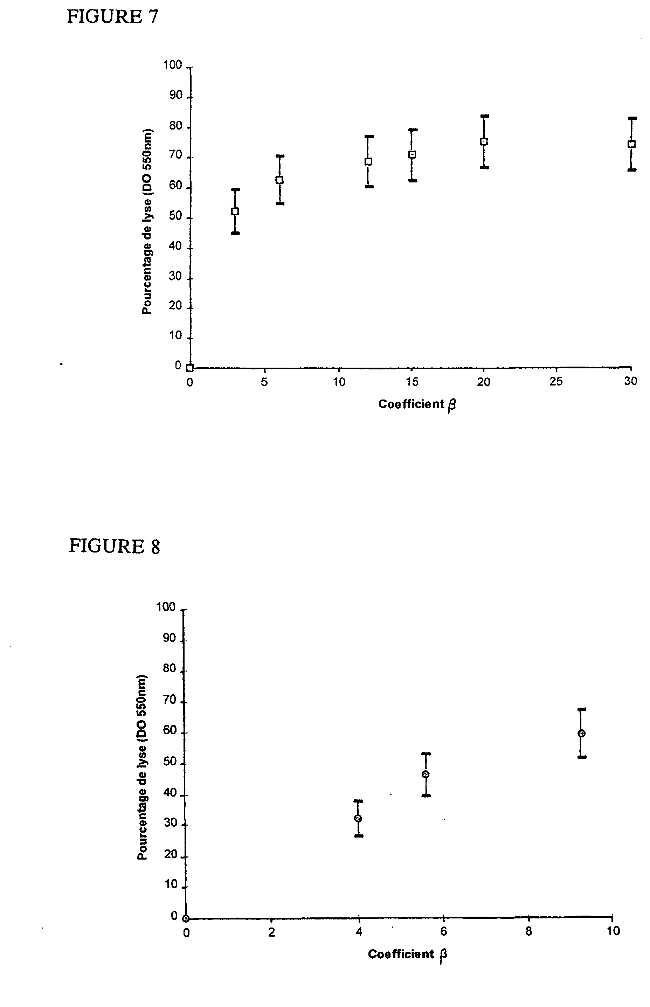

Une étude similaire a été réalisée en utilisant des tubes Falcon de polypropylène (Vc = 6ml) à la place des tubes Eppendorf. Une même valeur du coefficient α a été fixée (α = 3,3). Le volume de la suspension bactérienne a varié entre 200 μl et 2ml. β a varié de 3 à 30. La figure 7 illustre le pourcentage de lyse obtenu en fonction du coefficient β. La lyse de *S. epidermidis est* plus efficace pour des valeurs de β comprises entre 6 et 30 : le pourcentage de lyse est supérieur à 50%. Pour des valeurs de β > 30, le volume de l'échantillon est trop faible pour permettre une récupération complète du volume initial de la suspension cellulaire, pour α = 3,3. De préférence, l'efficacité de la lyse cellulaire est meilleure pour des valeurs de β comprises entre 12 et 30 : le pourcentage de lyse est supérieur à 60%.

-forme disque

Le protocole de lyse a été réalisé avec le même contenant utilisé dans l'exemple 1 (diamètre des puits 30 mm, vc = 2,8 ml), avec une valeur de coefficient α (α = 3,3) pendant deux minutes. Le volume de la suspension bactérienne a varié de 300 à 700 μl/puits. Le coefficient β a varié de 4 à 9,3. Après l'étape de vortex, le pourcentage de lyse des échantillons recueillis a été analysé par mesure de densité optique à 550 nm, les acides nucléiques libérés on été observés sur gel d'agarose et quantifiés par analyse Vidas.

Sur la figure 8 est indiqué le pourcentage de lyse obtenu en fonction du coefficient β. La lyse de *S.epidermidis* est efficace en contenant " format carte " pour des valeurs de β supérieures ou égales à 9,3. Lorsque β est égal à 9,3 avec une valeur non optimale de α (α = 3,3), 60% des cellules sont lysées. l'analyse sur gel d'agarose 0,8% du lysat montre que les profils de migration des acides nucléiques libérés ne sont pas modifiés quelles que soient les valeurs de β et sont similaires à ceux décrits pour le format tube. la quantité d'acides nucléiques mesurée par analyse Vidas est corrélée au pourcentage de lyse pour chaque essai.

Exemple 3 : Influence de la géométrie du tube sur l'efficacité de la lyse par vortex

Le protocole de lyse a été réalisé dans des contenants de différentes géométries et différents volumes (Vc), en présence de 300 μl de suspension cellulaire et 90 μl de billes de diamètre 100 μm pendant 2 minutes (soit α = 3,3 et β variable).

A : tube Eppendorf en polypropylène à fond en forme de V, Vc = 1,5 ml
B : cuvette de spectrométrie en polypropylène à fond plat, Vc = 5 ml
C : tube à fond plat, Vc = 30 ml
D : tube à fond plat, Vt = 60 ml
E = bouteille de verre à fond plat, Vc = 8 ml
F = bouteille de verre à fond plat, Vc = 25 ml
G = tube en polystyrène à fond en forme de U, Vc = 6 ml
H = tube en polystyrène à fond en forme de U, Vc = 14 ml
I = tube en polystyrène à fond en forme de U, Vc = 22 ml
J = tube en polypropylène à fond en forme de U, Vc = 6 ml
K = tube en polypropylène à fond en forme de U, Vc = 14 ml
L = tube en polypropylène à fond en forme de U, Vc = 22 ml
M = tube en polypropylène à fond en forme de U, Vt = 4 ml.

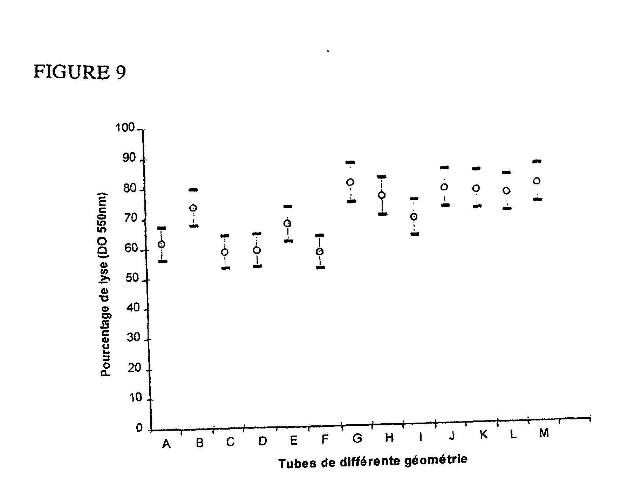

La figure 9 indique le pourcentage de lyse obtenu en fonction de la géométrie du contenant utilisé. Le pourcentage de lyse est compris en moyenne entre 60 et 75% avec les tubes à fond plat ou à fond en forme de V, par contre il est compris en moyenne entre 70 et 80% avec les tubes à fond en forme de U pour un même temps de vortex et une même valeur de α. La mesure par analyse Vidas de la quantité d'acides nucléiques libérés confirme cette influence.

Exemple 4 : influence de l'ajout de billes de plus gros diamètre sur l'efficacité de la lyse par vortex

Le protocole de lyse a été réalisé en tube Eppendorf, en présence de 300 µl de suspension cellulaire et 90 µl de billes de diamètre 100 µm (soit α = 3,3 et β = 5) pendant deux minutes. Différentes billes on été ajoutées au milieu en combinaison : billes de fer de diamètre 2 mm, billes de verre de diamètre 3 mm.

La figure 10 présente le pourcentage de lyse de *S.epidermidis* en fonction des différentes combinaisons de billes ajoutées. L'addition de bille de fer ou de billes de verre entraine une augmentation du pourcentage de lyse. Ce pourcentage est sensiblement meilleur en présence de jusqu'à 10 billes/tube. la présence de ces 10 billes ne gêne pas le mouvement des billes de diamètre 100 µm; par contre au delà de cette valeur, le mouvement est gêné. Le pourcentage de lyse obtenu est plus élevé en présence d'un même nombre de billes de verre par essai que de billes de fer. D'après la figure 11, l'addition de combinaison de billes de fer et de verre permet d'obtenir un pourcentage de lyse aussi élevés qu'en présence de l'addition d'un mélange homogène de billes pour chaque combinaison testée; le pourcentage de lyse est supérieur à 80%. L'analyse sur gel d'agarose 0,8% des acides nucléiques libérés montre que les molécules d'ADN et d'ARNr ont le même profil de migration après lyse quelles que soient les combinaisons des billes utilisées. L'addition des billes de fer et de verre n'altère pas la structure du matériel nucléique libéré. De plus il a été vérifié que leur présence dans le lysat ne perturbe pas les réactions d'amplification PCR et NASBA.

Exemple 5 : Influence du temps de vortex sur l'efficacité de la lyse par vortex

Trois protocoles de lyse ont été réalisés en parallèle :

- Protocole I : tube Eppendorf Vc = 1,5 ml, 90 µl de bille de diamètre 100 µm et 300 µl de suspension cellulaire α = 3,3 et β = 5).
- Protocole II : protocole I avec addition de 3 billes de verre de diamètre 3 mm et 5 billes de fer de diamètre 2 mm.
- Protocole III : protocole II en tube Falcon (Vc = 6ml) à la place des Eppendorfs.

Pour chaque protocole, différents temps de vortex ont été réalisés.

La figure 12 présente le pourcentage de lyse obtenu en fonction du temps de vortex, pour les protocoles I et II. Les conditions expérimentales du protocole I ne sont pas optimales. On confirme qu'après 2 minutes de vortex, seulement 60% des bactéries S. *epidermidis* sont lysées. Par contre il est possible d'améliorer l'efficacité du protocole malgré ces conditions non optimales en augmentant le temps de vortex : dès 8 minutes de vortex, 85% des bactéries sont lysées. Les conditions expérimentales du protocole II sont plus optimales que celles du protocole I, dû à l'addition de billes de diamètre 3 mm : dans ces conditions, 85% des bactéries S. *epidermidis* sont lysées dès 4 minutes de vortex.

La figure 13 présente le pourcentage de lyse obtenu en fonction du temps de vortex pour les protocoles II et III. Les conditions expérimentales du protocole III sont plus optimales que celles du protocole II : 88% des bactéries S. *epidermidis* sont lysées après seulement 2 minutes de vortex alors que seulement 80% ou 60% des bactéries après 2 minutes avec le protocole II ou I, respectivement. La mesure par analyse Vidas des acides nucléiques libérés confirme ces principales observations.

Ainsi, augmenter le temps de vortex permet d'obtenir un pourcentage de lyse élevé lorsque les conditions expérimentales initiales ne sont pas optimales .Parallèlement, lorsque ces conditions sont optimales, le temps de vortex peut être court (2 minutes).

Exemple 6 : Universalité du protocole de lyse selon l'invention

Le procédé de lyse selon l'invention peut être appliqué à d'autres espèces cellulaires. Pour cela, le protocole a été réalisé en tube Eppendorf en présence de 300 µl de suspension cellulaire de concentration $1.10^9$ cellules /ml, de 90 µl de billes de diamètre 100 µm avec un temps de vortex égal à 8 minutes. Différentes cellules ont été respectivement utilisées *(Mycobacterium gordonae, Escherichia coli, Listeria monocytogenes, Streptococcus pneumonies, Bacillus stearothermophilus, Micrococcus sp., Actinomyces viscosus, Actinomyces naeslundii, Stomatococcus mucilaginosus, Nocardia asteroides, Rhodococcus sp.).* Les résultats obtenus indiquent que le pourcentage de lyse pour chacun des cas analysés est compris entre 80 et 90%. Les profils de migration sur gel d'agarose 0,8% des acides nucléiques libérés dans chaque lysat sont similaires pour chaque souche bactérienne testée, et sont similaires à celui d'ADN et ARNR 16S *d'E. coli* purifiés et commercialisés.

Exemple 7 : Lyse de levure

Espèce testée: Candida albicans
Contenant: Tube Falcon en polypropylène de Vc = 6 ml.
Echantillon ajusté à 0.5 McFarland, soit 3.106 levures/ml.
Volume échantillon: Ve = 600 µl
Volume apparent des billes: Vb (µl, variable): 60, 90 et 180 µl.
Diamètre des billes en verre testées (µ):100, 500et 1500 µm.
L'efficacité de lyse est mesurée d'après le pourcentage de lyse:
% de lyse = DO550 (avant lyse) - DO550 (aprrès lyse) / DO550 (avant lyse
Durée du vortex: 2 à 20 mn

| Résultats | | | |
|---|---|---|---|
| Diamètre billes | (µ)Vb (µl) | Durée du vortex (mn) | % de lyse |
| 100 | 90 | 2 | 0 |
| | | | |
| 100 | 90 | 8 | 0 |
| | | | |
| 1500 | 90 | 2 | 0 |
| | | | |
| 1500 | 90 | 8 | 30 |
| | | | |
| 1500 | 90 | 20 | 36 |
| | | | |

On démontre qu'un diamètre de 100 µm est insuffisant.

| Diamètre billes (µ) | Vb (µl) | Durée du vortex (mn) | % de lyse |
|---|---|---|---|
| 1500 | 180 | 8 | 30 |
| 1500 | 180 | 20 | 29 |
| 1500 | 60 | 8 | 33 |

On démontre qu'avec un diamètre de billes de 1500 µm, on atteint un pourcentage de lyse d'environ 30%, pour un Vb compris entre 60 et 180 et avec une durée de vortex entre 8 et 20mn.

| Diamètre billes (µ) | Vb (µl) | Durée du vortex (mn) | % de lyse |
|---|---|---|---|
| 500 | 60 | 8 | 52 |
| 500 | 60 | 20 | 58 |
| 500 | 180 | 20 | 71 |

On démontre qu'un diamètre de 500µ permet d'obtenir un pourcentage de lyse de plus de 50%, avec un Vb de 60 à 180µl et une durée de vortex de 8 à 20mn.
Conclusion:
Des billes de 500 µm de diamètre, avec une durée de vortex comprise entre 8 et 20 mn sont nécessaires comme paramètre en combinaison avec le mouvement de type Vortex pour lyser des levures.

Exemple 8 : Sensibilité du protocole de lyse selon l'invention

Les acides nucléiques ADN et ARNr 16S libérés par lyse selon le procédé de l'invention ont été soumis à une amplification. Les conditions de lyse sont les mêmes que celles indiquées dans l'exemple 6. La souche bactérienne utilisée est *S. epidermidis*. Deux protocoles d'amplification spécifiques des acides nucléiques de *S. epidermidis* ont été réalisés à partir des lysats recueillis, soit un protocole PCR pour l'amplification de l'ADN, soit une protocole NASBA pour l'amplification de l'ARNr 16S, comme décrits ci-dessus dans le protocole test général.

1)- Amplification PCR de l'ADN libéré

Des concentrations initiales de 1. $10^9$ à 1. $10^2$ cellules /ml ont été utilisées. 10 µl de lysat ont été utilisés pour chaque essai PCR. Les amplicons produits ont été détectés et quantifiés après hybridation sandwich spécifique sur Vidas, comme décrit dans le protocole test général. Les amplicons produits sont mesurables pour des concentrations bactériennes initiales au moins supérieures ou égales à $1.10^2$ cellules /ml, soit 30 cellules /300 µl. Ainsi le protocole de lyse optimisé par vortex est efficace pour lyser *au moins* 30 cellules /300 µl et pour libérer des molécules d'ADN amplifiables par PCR de sorte que la quantité d'amplicons produits soit détectables.

2)- Amplification NASBA de l'ARNr 16S

Des concentrations initiales de $2.10^9$ à $2.10^2$ cellules /ml ont été utilisées. 5 µl de lysat ont été utilisés pour chaque essai NASBA. Les amplicons produits ont été détectés et quantifiés sur microplaque par hybridation sandwich avec des sondes oligonucléotidiques spécifiques de *S. epidermidis*, comme décrit dans le protocole test général. Les amplicons produits ont pû être détectés et mesurés pour des concentrations bactériennes initiales *au moins* supérieures ou égales à $2.10^2$ cellules /ml, soit 60 cellules /300 µl. La quantité d'amplicons mesurée est importante pour cette concentration initiale, ce qui indique que probablement des amplicons produits après lyse de bactéries de concentration initiale inférieure à 60 cellules /300 µl seraient détectables et mesurables.

Exemple 9 : Résolution de la formation de mousse issue de l'agitation de billes dans un échantillon.

A des fins d'automatisation, il est capital de résoudre et d'empêcher la formation de mousse dans les liquides, ce qui perturbe voire empêche les étapes de transfert par pipettage. Des agents " antimousse " ont été étudiés quant à leur capacité à limiter ce type de phénomène. Pour ce faire, un protocole a été validé à partir d'échantillons respiratoires (crachats, expectorations, lavages broncho-alvéolaires) fluidifiés et inactivés selon le protocole "NaLc". Brièvement, 2 ml d'échantillon sont ajoutés à 2 ml de solution antiseptique (obtenue par dissolution de 0,75 g de N-actétyl-L-Cystéine dans 150 ml de soude 4%). Le mélange est vortexé et laissé sous agitation pendant 20 minutes à température ambiante, puis neutralisé par 41 ml de tampon phosphate (pH 6,8). L'ensemble est vortexé 15 secondes puis centrifugé à 4°C pendant 25 minutes à 4000 g. Le culot est resupendu (vortex, 15 secondes) dans 2 ml residuels après avoir éliminé le surnageant.

Différents échantillons sont ensuite mélangés afin de disposer d'un milieu homogène et constant pour l'étude. Les essais sont réalisés de la manière suivante : 260 µl d'échantillons sont ajoutés dans un tube polypropylène 12 x 75 mm avec 90 µl de billes de verre de diamètre 100 µm, 3 billes de verre de diamètre 2 mm, 5 billes de fer de diamètre 2 mm. 40 µl d'un détergent anionique sont ajoutés afin d'obtenir une concentration finale de 2,5 %. La présence d'un détergent est justifiée par le fait de stabiliser et de protéger les acides nucléiques lors du processus de lyse.

L'ensemble est agité dans un vortex Reax 2000 (Heidolph) à la puissance maximale pendant 12 minutes (temps particulièrement long par rapport au protocole standard, mais qui doit favoriser la formation éventuelle de mousse le cas échéant). La hauteur du liquide dans le tube est mesurée initialement puis après agitation et le pourcentage de mousse obtenu est exprimé dans les résultats suivants en faisant le rapport de la hauteur de mousse sur la hauteur initiale.

Tableau N

| Concentration antimousse (%) | Génération de mousse (%) |
| --- | --- |
| 0 | 22 % |
| 0.01 | 11 % |
| 0.03 | 11 % |
| 0.1 | 11 % |
| 0.5 | 0 % |
| 1 % | 0 % |

L'agent antimousse, en particulier le Foam Ban MS-575 (Ultra Inc., Charlotte, USA) est ajouté sous un volume négligeable, afin d'obtenir des concentrations finales de 0,01 à 1 % (*Tableau N*).

Les résultats (voir *Tableau N*) montrent que la présence d'un antimousse permet la résolution de la formation de mousse, notamment à partir d'échantillons cliniques tels que les sédiments. Son utilisation à une concentration de 0,5 à 1 % permet de résoudre totalement la formation de mousse issue de l'agitation de l'échantillon liquide en présence de billes.

L'efficacité de l'antimousse a également été vérifiée à partir d'échantillons individuels (non mélangés), à la concentration finale de 0,6 %, selon le protocole décrit précédemment.

Les résultats (voir Tableau N+1) montrent que la genèse de mousse est variable selon les échantillons biologiques, mais que la présence d'antimousse au sein du protocole de lyse, résout et empêche la formation de mousse à partir de différents échantillons respiratoires.

Tableau N + 1

| Echantillon | Génération de mousse (%) | |
|---|---|---|
| | avec mousse | sans mousse |
| 2675 6/2 | 0% | 16% |
| 2677 6/2 | 0% | 16% |
| 2654 6/2 | 0% | 16% |
| 2673 6/2 | 0% | 22% |
| 3609 71 | 0% | 11% |
| 3673 92 | 0% | 16% |

Exemple 10 :Sensibilité du protocole de lyse de microorganismes en présence d'agents antimousse

L'efficacité de l'ensemble du protocole de lyse, notamment en présence d'antimousse, a été évaluée sur des échantillons contenant de très faibles concentrations de bactéries. La bactéries *Mycobacterium bovis* BCG a été utilisée, car le genre *Mycobacterium* est connu comme étant difficile à lyser, et particulièrement recherché dans les prélèvements respiratoires.

Des dilutions en cascade, à partir d'une suspension initiale homogène de bactéries dont la concentration a été déterminée par mesure densitométrique (Densité Optique à 550 nm) ont été réalisées afin d'inoculer sous un volume négligeable des fractions de sédiment négatif (reconstitué par mélange de différents sédiments négatifs fluidifiés et décontaminés) inactivé (chauffage à 95°C pendant 15 minutes). Les échantillons ainsi reconstitués ont été soumis au protocole suivant :

· dilution dans un tampon contenant un détergent anionique et de l'antimousse Foam Ban MS-575 pour obtenir des concentrations de 2,5 % et 1% final, respectivement.

· chauffage à 95°C pendant 15 mn (inactivation des bactéries).

· un volume de 600 µl est ajouté dans la cuve 12 x 75 mm (polypropylène) contenant 180 µl de billes de verre de 100 µm, 6 billes de verre (diamètre 2 mm), 10 billes de fer (diamètre 2 mm) et l'ensemble est agité à l'aide d'un vortex pendant 2 minutes à la puissance maximale.

En parallèle, des témoins d'expériences sont réalisés : ceux-ci subissent le même protocole que précédemment décrit hormis l'étape de d'agitation des billes par vortex.

Les acides nucléiques ainsi potentiellement libérés sont alors purifiés par une étape de capture spécifique sur des billes magnétiques (Seradyne) dérivées avec un oligonucléotide de capture complémentaire de la séquence de l'ARN 16S de *M. tuberculosis* BCG, selon le protocole suivant :

· 250 µl de lysat obtenu sont mélangés à un tampon de capture contenant les sondes oligonucléotidiques couplées à des billes magnétiques (50 µg/essai). L'ensemble est incubé 20 mn à 60°C, puis 10 mn à température ambiante, puis le surnageant est éliminé après immobilisation des billes sur les parois du tube par aimantation.

· les billes sont lavées (resuspension puis aimantation) par 2 fractions de 1 ml de tampon de lavage.

· après élimination du tampon, les billes sont resuspendues par 50 µl d'eau et 25 µl du mélange d'amplification contenant des amorces oligonucléotidiques et des désoxyribonucléotides de la trousse d'identification *de M. tuberculosis* ("Amplified Mycobacterium Tuberculosis Direct", Gen-Probe ref. 1001), qui est basé sur la "Transcription-Mediated Amplification" (TMA), décrite par McDonough et coll. (Nucleic Acids Amplification Technologies, 1997, Ed. Lee, Morse & Olsvik, Eaton Publishing, Natik, USA, pp. 113-123) ainsi que Kacian et Fulz (Nucleic Acid Sequences Amplification Methods, 1995, Brevet des Etats Unis d'Amérique, N; 5,399,491).

· l'ensemble est chauffé à 60°C pendant 15 mn, refroidi à 42°C pendant 5 mn, et 25 µl de la solution enzymatique de la trousse (contenant de l'ARN polymérase T7 et de la reverse-transcriptase) sont ajoutés.

· la réaction est incubée pendant 1 heure à 42°C, puis analysée en utilisant les réactifs de détection de la trousse, suivant la technique "Hybridization Protection Assay" (HPA) décrite par Arnold et coll. (Clin. Chem., 1989, vol. 35, pp. 1588-1594).

· Les réactions sont ensuites lue sur un luminomètre 450 i (Gen-Probe) et aboutissent à des données en unités de luminescence relatives (RLU), la limite de positivité d'un essai étant 30000 RLUs.

Les données obtenues sont indiquées dans le tableau N+2, et correspondent a trois essais expérimentaux par conditions testées.

Tableau N + 2

| Bactéries/ essai | 10 Exp - 3 | 10 Exp - 1 | 10 Exp + 1 | 10 Exp + 3 | |
|---|---|---|---|---|---|
| Sans Vortex | 415 | 402 | 508 | 119736 | |
| | 467 | 554 | 422 | 2312 | |
| | 371 | 364 | 179756 | 1013140 | |
| | 418 | 440 | 60229 | 378396 | Moyenne |
| | 48 | 101 | 103514 | 552831 | Ecart Type |
| | 12 | 23 | 172 | 146 | Coefficient de variation (%) |
| Avec Vortex | 43117 | 457 | 1029901 | 1079399 | |
| | 1088 | 919609 | 125 | 1030602 | |
| | 389 | 453 | 1069273 | 1058371 | |
| | 144198 | 306840 | 700100 | 1056124 | Moyenne |
| | 248479 | 530674 | 605650 | 24476 | Ecart Type |
| | 172 | 173 | 87 | 2 | Coefficient de variation (%) |

Les résultats (voir *Tableau N+2*) montrent que la réalisation du protocole de lyse par billes de verre en présence d'agents antimousse permet de détecter dans les échantillons biologiques une quantité équivalente à $10^{-3}$ bactéries/essai, soit une quantité équivalente à 1 à 10 copies de ribosomes de manière sensible. En comparaison, l'absence de lyse (sauf chauffage initial en présence de détergent pour l'inactivation des échantillons) ne peut détecter que l'équivalent de 10 à 1000 bactéries par essai, soit 10 000 fois moins qu'avec la méthode de lyse utilisée.

Ces résultats montrent la validité du protocole associant la lyse de bactéries par choc mécanique en présence de billes (à l'aide d'un vortex ou autre) combinée à l'utilisation d'agents antimousse. La combinaison d'un procédé de lyse par agitation de billes est donc parfaitement adaptée.

**Revendications**

1.  Procédé de lyse d'un échantillon biologique comprenant au moins un micro-organisme de type bactérie, pour libérer au moins un matériel nucléique d'intérêt appartenant audit micro-organisme, selon lequel:

    -   on dispose dans un contenant ledit échantillon biologique en milieu liquide,
    -   on dispose dans ledit contenant au moins un matériau particulaire, relativement dur, et substantiellement inerte par rapport au matériel nucléique,
    -   on soumet le mélange de l'échantillon biologique et du matériau particulaire à un mouvement,

        **caractérisé en ce que**, en combinaison:

le mouvement choisi est de type vortex, et répond aux conditions suivantes:

- le matériau particulaire est constitué de billes de diamètre compris entre 90 et 150 $\mu$m, et
- le volume apparent des billes , Vb, et le volume de l'échantillon liquide, Ve, sont liés par la relation Ve = $\alpha$.Vb, avec $\alpha$ compris entre 1,4 et 10 lorsque le contenant est de forme tubulaire, et $\alpha$ inférieur ou égal à 2,1 lorsque le contenant est de forme disque,

moyennant quoi, sans ajout de réactif et/ou étape opératoire supplémentaire, on libère directement dans le milieu liquide le matériel nucléique à l'état natif et accessible à tout réactif de traitement postérieur.

2. Procédé de lyse d'un échantillon biologique comprenant au moins un micro-organisme de type levure, pour libérer au moins un matériel nucléique d'intérêt appartenant audit micro-organisme, selon lequel:

- on dispose dans un contenant ledit échantillon biologique en milieu liquide,
- on dispose dans ledit contenant au moins un matériau particulaire, relativement dur, et substantiellement inerte par rapport au matériel nucléique,
- on soumet le mélange de l'échantillon biologique et du matériau particulaire à un mouvement,

   **caractérisé en ce que**, en combinaison:

le mouvement choisi est de type vortex, et répond aux conditions suivantes:

- le matériau particulaire est constitué de billes ayant un diamétre de 500 $\mu$m, et
- le volume apparent des billes, Vb, et le volume de l'échantillon liquide, Ve, sont liés par la relation Ve = $\alpha$.Vb, avec $\alpha$ compris entre 1,4 et 10 lorsque le contenant est de force tubulaire, et $\alpha$ inférieur ou égal à 2,1 lorsque le contenant est de forme disque,

moyennant quoi, sans ajout de réactif et/ou étape opératoire supplémentaire, on libère dans le milieu liquide le matériel nucléique à l'état natif et accessible à tout réactif de traitement postéreur.

3. Procédé selon la revendication 1, **caractérisé en ce que** le matériau particulaire est constitué de billes ayant un diamètre de 100 $\mu$m.

4. Procédé selon les revendications 1 ou 2, **caractérisé en ce que** le mouvement de type vortex répond en outre à la relation suivante:

$$Vb < Ve < Vc,$$

selon laquelle

$$Vc = \beta.Ve,$$

et

$\beta$ est supérieur ou égal à 2,5, Vc étant le volume disponible du contenant.

5. Procédé selon la revendication 1 ou 2, **caractérisé en ce que** le contenant est de forme tubulaire, de préférence à fond en forme de U.

6. Procédé selon la revendication 4, **caractérisé en ce que** $\beta$ est un nombre compris entre 2,5 et 30.

7. Procédé selon les revendications 1 ou 2, 4 et 5, **caractérisé en ce que** le contenant est un tube à fond tronconique, et $\alpha$ est un nombre compris entre 1,4 et 3,3, et $\beta$ est un nombre compris entre 2,5 et 15, de préférence entre 3,75 et 15.

8. Procédé selon les revendications 1 ou, 4 et 2 et 5, **caractérisé en ce que** le contenant est un tube à fond en forme de U, et $\alpha$ est un nombre compris entre 1,4 et 3,3, de préférence est égal à 3,3, et $\beta$ est un nombre compris entre 3 et 30, de préférence entre 12 et 30, par exemple égal à 20.

9. Procédé selon la revendication 1 ou 2, et 4 **caractérisé en ce que** le contenant est sous forme disque et $\alpha$ est inférieur ou égal à 1,4 et $\beta$ est supérieur ou égal à 9,3.

10. Procédé selon la revendication 1 ou 2, **caractérisé en ce que** le matériau particulaire comprend d'autres billes de diamètre supérieur à celui desdites billes.

11. Procédé selon la revendication 10, **caractérisé en ce que** le contenant comprend jusqu'à y compris 16 autres billes, de préférence jusqu'à y compris 10 autres billes, ayant un diamètre égal à de 2 à 3 mm,.

12. Procédé selon la revendication 1 ou 2, **caractérisé en ce que** l'on ajoute une substance anti-mousse à une concentration finale de 0,01 à 1%, de préférence de 0,5 à 1% du volume de l'échantillon liquide, Ve.

13. Procédé selon la revendication 12, **caractérisé en ce que** l'on ajoute en outre un détergent de type anionique à une concentration finale de 2,5% du volume de l'échantillon liquide, Ve.

14. Procédé selcn la revendication 1 **caractérisé en ce que** le temps de mise en mouvement de type vortex est au moins égal à 10 secondes, de préférence au moins égal à 20 secondes, par exemple entre 1 et 5 minutes.

15. Procédé selon la revendication 14, **caractérisé en ce que** le temps de mise en mouvement de type vortex est de 2 minutes.

16. Procédé selon la revendication 2 **caractérisé en ce que** le temps de mise en mouvement de type vortex est compris entre 8 et 20 minutes.

17. Procédé de traitement d'un échantillon biologique comprenant au moins un micro-organisme de type bactérie ou levure, comportant un matériel nucléique d'intérêt, selon lequel:

    - on procède à une lyse dudit échantillon, selon le procédé selon l'une quelconque des revendications 1 à 16,
    - on soumet l'échantillon biologique lysé, avec ou sans le matériau particulaire ayant servi à la lyse, directement à un protocole opératoire concernant spécifiquement le matériel nucléique d'intérêt, par exemple amplification ou détection nucléique.


**Patentansprüche**

1. Verfahren zum Lysieren einer biologischen Probe, die zumindest einen Mikroorganismus vom bakteriellen Typ enthält, um zumindest ein zu dem genannten Mikroorganismus gehörendes, interessierendes Nukleinmaterial freizusetzen, bei dem

    - in einem Behälter die biologische Probe in einem flüssigen Milieu bereitgestellt wird,
    - in den Behälter zumindest ein partikelartiges Material gegeben wird, das verhältnismäßig hart und bezogen auf das Nukleinmaterial im Wesentlichen inert ist, und
    - die Mischung aus der biologischen Probe und dem partikelartigen Material einer Bewegung unterworfen wird,

      **dadurch gekennzeichnet, dass** in Kombination
      die gewählte Bewegung vom Vortex-Typ ist und folgenden Bedingungen entspricht:

    - das partikelartige Material besteht aus Kugeln mit einem Durchmesser, der zwischen 90 und 150 µm liegt, und
    - das apparente Volumen Vb der Kugeln und das Volumen Ve der flüssigen Probe erfüllen die Bedingung Ve = $\alpha$ Vb, wobei $\alpha$ zwischen 1,4 und 10 liegt, wenn der Behälter rohrförmig ist, und $\alpha$ kleiner oder gleich 2,1 ist, wenn der Behälter scheibenförmig ist,
      wodurch ohne Zugabe von Reagens und/oder einem ergänzenden Verfahrensschritt das Nukleinmaterial direkt in das flüssige Milieu freigesetzt wird, und zwar in einem nativen Zustand und zugänglich für jedes Reagens einer nachfolgenden Verarbeitung.

2. Verfahren zum Lysieren einer biologischen Probe, die zumindest einen Mikroorganismus vom Hefetyp enthält, um zumindest ein zu dem genannten Mikroorganismus gehörendes, interessierendes Nukleinmaterial freizusetzen, bei dem

- in einem Behälter die biologische Probe in einem flüssigen Milieu bereitgestellt wird,
- in den Behälter zumindest ein partikelartiges Material gegeben wird, das verhältnismäßig hart und bezogen auf das Nukleinmaterial im Wesentlichen inert ist, und
- die Mischung aus der biologischen Probe und dem partikelartigen Material einer Bewegung unterworfen wird,

**dadurch gekennzeichnet, dass** in Kombination
die gewählte Bewegung vom Vortex-Typ ist und folgenden Bedingungen entspricht:

- das partikelartige Material besteht aus Kugeln mit einem Durchmesser von 500 $\mu$m, und
- das apparente Volumen Vb der Kugeln und das Volumen Ve der flüssigen Probe erfüllen die Bedingung Ve = $\alpha$ Vb, wobei $\alpha$ zwischen 1,4 und 10 liegt, wenn der Behälter rohrförmig ist, und $\alpha$ kleiner oder gleich 2,1 ist, wenn der Behälter scheibenförmig ist,
wodurch ohne Zugabe von Reagens und/oder einem ergänzenden Verfahrensschritt das Nukleinmaterial direkt in das flüssige Milieu freigesetzt wird, und zwar in einem nativen Zustand und zugänglich für jedes Reagens einer nachfolgenden Verarbeitung.

3. Verfahren nach Anspruch 1, **dadurch gekennzeichnet, dass** das partikelartige Material aus Kugeln mit einem Durchmesser von 100 $\mu$m besteht.

4. Verfahren nach den Ansprüchen 1 oder 2, **dadurch gekennzeichnet, dass** die Bewegung vom Typ Vortex der folgenden Beziehung gehorcht:

$$Vb < Ve < Vc,$$

bei der

$$Vc = \beta\ Ve$$

und
$\beta$ größer oder gleich 2,5 ist, wobei Vc das verfügbare Volumen des Behälters ist.

5. Verfahren nach Anspruch 1 oder 2, **dadurch gekennzeichnet, dass** der Behälter rohrförmig ist, vorzugsweise mit einem Boden in Form eines U.

6. Verfahren nach Anspruch 4, **dadurch gekennzeichnet, dass** $\beta$ eine Zahl ist, die zwischen 2,5 und 30 liegt.

7. Verfahren nach den Ansprüchen 1 oder 2, 4 und 5, **dadurch gekennzeichnet, dass** der Behälter ein Rohr mit einem kegelstumpfartigen Boden ist und dass $\alpha$ eine Zahl ist, die zwischen 1,4 und 3,3 liegt, und dass $\beta$ eine Zahl ist, die zwischen 2,5 und 15, vorzugsweise zwischen 3,75 und 15 liegt.

8. Verfahren nach den Ansprüchen 1 oder 4 und 2 und 5, **dadurch gekennzeichnet, dass** der Behälter ein Rohr mit einem Boden in Form eines U ist, und dass $\alpha$ eine Zahl ist, die zwischen 1,4 und 3,3 liegt und vorzugsweise gleich 3,3 ist, und dass $\beta$ eine Zahl ist, die zwischen 3 und 30, vorzugsweise zwischen 12 und 30 liegt und beispielsweise gleich 20 ist.

9. Verfahren nach Anspruch 1 oder 2, und 4, **dadurch gekennzeichnet, dass** der Behälter scheibenförmig ist und dass $\alpha$ kleiner oder gleich 1,4 ist und $\beta$ größer oder gleich 9,3 ist.

10. Verfahren nach Anspruch 1 oder 2, **dadurch gekennzeichnet, dass** das partikelartige Material weitere Kugeln mit einem Durchmesser enthält, der größer als der Durchmesser der genannten Kugeln ist.

11. Verfahren nach Anspruch 10, **dadurch gekennzeichnet, dass** der Behälter einen Durchmesser von gleich 2 mm bis gleich 3 mm hat, solange er sechzehn andere Kugeln, vorzugsweise solange er zehn andere Kugeln enthält.

**12.** Verfahren nach Anspruch 1 oder 2, **dadurch gekennzeichnet, dass** ein Antischaum-Mittel in einer Endkonzentration von 0,01 bis 1 %, vorzugsweise von 0,5 bis 1 % vom Volumen Ve der flüssigen Probe zugegeben wird.

**13.** Verfahren nach Anspruch 12, **dadurch gekennzeichnet, dass** unter anderem ein anionisches Detergens in einer Endkonzentration von 2,5 % des Volumens Ve der flüssigen Probe zugegeben wird.

**14.** Verfahren nach Anspruch 1, **dadurch gekennzeichnet, dass** die Zeitspanne für die Ausübung der Bewegung vom Typ Vortex wenigstens gleich 10 Sekunden, vorzugsweise wenigstens gleich 20 Sekunden ist und beispielsweise zwischen 1 und 5 Minuten liegt.

**15.** Verfahren nach Anspruch 14, **dadurch gekennzeichnet, dass** die Zeit zur Durchführung der Bewegung vom Typ Vortex 2 Minuten beträgt.

**16.** Verfahren nach Anspruch 2, **dadurch gekennzeichnet, dass** die Zeit zur Durchführung der Bewegung vom Typ Vortex zwischen 8 und 20 Minuten liegt.

**17.** Verfahren zur Behandlung einer biologischen Probe, die zumindest einen Mikroorganismus vom Bakterien- oder Hefetyp enthält, der ein interessierendes Nukleinmaterial enthält, bei dem

- eine Lyse der genannten Probe gemäß dem Verfahren nach einem der Ansprüche 1 bis 16 durchgeführt wird,
- die lysierte biologische Probe mit oder ohne dem partikelartigen Material, das der Lyse gedient hat, direkt einem Verarbeitungsprotokoll unterworfen wird, das spezifisch das interessierende Nukleinmaterial betrifft, beispielsweise einer Nuklein-Amplifikation oder -Detektion.

**Claims**

**1.** Lysis method of a biological specimen comprising at least one bacteria type micro-organism to release at least one nucleic substance of interest belonging to said micro-organism, wherein :

- said biological specimen is placed in a container in liquid medium,
- at least one relatively hard particulate substance substantially inert with respect to the nucleic substance is placed in said container,
- the mixture of the biological specimen and the particulate substance is subjected to a movement,

 **characterised in that**, in combination :

 the selected movement of the vortex type, and meets the following conditions :

- the particulate substance consists of beads between 90 and 150 µm in diameter, and
- the apparent volume of the beads, Vb, and the volume of the liquid specimen, Ve, are linked by the equation Ve = $\alpha$.Vb, where $\alpha$ is between 1.4 and 10 when the container is tubular in shape, and $\alpha$ is less than or equal to 2.1 when the container is disk-shaped,

 by means of which, without addition of reagent and/or an additional operating step, the nucleic substance is released directly into the liquid medium in the native state, accessible to any subsequent processing reagent.

**2.** Lysis method of a biological specimen comprising at least one yeast type micro-organism to release at least one nucleic substance of interest belonging to said micro-organism, wherein :

- said biological specimen is placed in a container in liquid medium,
- at least one relatively hard particulate substance substantially inert with respect to the nucleic substance is placed in said container,
- the mixture of the biological specimen and the particulate substance is subjected to a movement,

 **characterised in that**, in combination :

 the selected movement of the vortex type, and meets the following conditions :

- the particulate substance consists of beads 500 μm in diameter, and
- the apparent volume of the beads, Vb, and the volume of the liquid specimen, Ve, are linked by the equation Ve = $\alpha$.Vb, where $\alpha$ is between 1.4 and 10 when the container is tubular in shape, and $\alpha$ is less than or equal to 2.1 when the container is disk-shaped,

by means of which, without addition of reagent and/or an additional operating step, the nucleic substance is released directly into the liquid medium in the native state, accessible to any subsequent processing reagent.

3. Method according to claim 1, **characterised in that** the particular substance consists of beads 100 μm in diameter.

4. Method according to claims 1 or 2, **characterised in that** the vortex type movement also complies with the following equation :

$$Vb < Ve < Vc,$$

wherein

$$Vc = \beta.Va,$$

and
$\beta$ is greater than or equal to 2.5, where Vc is the available volume of the container.

5. Method according to claim 1 or 2, **characterised in that** the container is tubular in shape, preferentially with a U-shaped bottom.

6. Method according to claim 4, **characterised in that** $\beta$ is a number between 2.5 and 30.

7. Method according to claims 1 or 4 and 2 and 5, **characterised in that** the container is a tapered bottomed tube, and $\alpha$ is a number between 1.4 and 3.3, and $\beta$ is a number between 2.5 and 15, preferentially between 3.75 and 15.

8. Method according to claims 1 or 2, 4 and 5, **characterised in that** the container is U-shaped bottomed tube, and $\alpha$ is a number between 1.4 and 3.3, preferentially equal to 3.3, and $\beta$ is a number between 3 and 30, preferentially between 12 and 20, for example equal to 20.

9. Method according to claim 1 or 2, and 4, **characterised in that** the container is disk-shaped and $\alpha$ is less than or equal to 1.4 and $\beta$ is greater than or equal to 9.3.

10. Method according to claim 1 or 2, **characterised in that** the particulate substance comprises other beads of a greater diameter than that of said beads.

11. Method according to claim 10, **characterised in that** the container comprises up to and including 16 other beads, preferentially up to and including 10 other beads, having a diameter equal to 2 to 3 mm.

12. Method according to claim 1 or 2, **characterised in that** an anti-foaming substance is added at a final concentration of 0.01 to 1%, preferentially 0.5 to 1% of the volume of the liquid specimen, Ve.

13. Method according to claim 12, **characterised in that** an anionic type detergent is also added at a final concentration of 2.5% of the volume of the liquid specimen, Ve.

14. Method according to claim 1, **characterised in that** the vortex type movement time is at least equal to 10 seconds, preferentially at least equal to 20 seconds, for example between 1 and 5 minutes.

15. Method according to claim 14, **characterised in that** the vortex type movement time is 2 minutes.

16. Method according to claim 2, **characterised in that** the vortex type movement time is between 8 and 20 minutes.

**17.** Processing method of a biological specimen comprising at least one bacteria or yeast type micro-organism, comprising a nucleic substance of interest, wherein:

- lysis of said specimen is performed, using the method according to any of claims 1 to 16,
- the lysed biological specimen is subjected, with or without the particulate substance used for the lysis, directly to an operating protocol related specifically to the nucleic substance of interest, for example nucleic amplification or detection.

FIGURE 1

FIGURE 2

FIGURE 3

FIGURE 4

FIGURE 5

FIGURE 6

FIGURE 7

FIGURE 8

FIGURE 9

FIGURE 10

FIGURE 11

Différentes combinaisons de billes /essai

FIGURE 12

FIGURE 13